# EUROPEAN PATENT SPECIFICATION

(11) **EP 0 561 885 B1**
(45) Date of publication and mention of the grant of the patent: **16.02.2000**
(21) Application number: 92900509.8
(22) Date of filing: 12.12.1991
(51) Int. Cl.: C07K 7/06, C07K 7/08, C07K 14/025, A61K 39/12

(54) **SUBUNIT PAPILLOMAVIRUS VACCINE**
PAPILLOMAVIRUSUNTEREINHEIT-IMPFSTOFF
VACCIN A SOUS-UNITES CONTRE LE VIRUS DU PAPILLOME

(30) Priority: 12.12.1990 AU 387690
(43) Date of publication of application: 29.09.1993
(73) Proprietor: THE UNIVERSITY OF QUEENSLAND, St Lucia, QLD 4072 (AU); CSL LIMITED, Parkville, VIC 3052 (AU)
(72) Inventor: TINDLE, Robert, Kenmore, QLD 4069 (AU); FERMANDO, Germain, Jamboree Heights, QLD 4074 (AU); FRAZER, Ian, St Lucia, QLD 4067 (AU)
(74) Representative: Goddar, Heinz J., Dr.
(86) International application number: AU9100575
(87) International publication number: WO9210513

(56) References cited:
- EP-A- 0 375 555
- EP-A- 0 451 550
- EP-A- 0 523 391
- AU-A- 7 351 591
- JOURNAL OF GENERAL VIROLOGY vol. 71, no. 11 , November 1990 , READING pages 2709 - 2717 MULLER ET AL. 'Identification of seroreactive regions of the human papillomavirus type 16 proteins E4, E6, E7 and L1.'
- JOURNAL OF GENERAL VIROLOGY vol. 71, no. 6 , June 1990 , READING pages 1347 - 1354 TINDLE ET AL. 'Identification of B epitopes in human papillomavirus type 16 E7 open reading frame protein'
- International Journal of Cancer, Volume 46, No. 4, issued October 1990 pages 703-711, J DILLNER, "Mapping of Linear Epitopes of Human Papillomavirus Type 16: The E1, E2, E4, E5, E6 and E7 Open Reading Frames", see whole document especially Table 1.
- Journal of General Virology, Volume 71, No. 11, issued November 1990, 1990, pages 2719-2724, V KRCHNAK et al., "Synthetic Peptides Derived from E7 Region of Human Papillomavirus type 16 used as Antigens in ELISA", see especially page 2722 column 2 to page 2723 column 2.
- Journal of Virology, Volume 64, No. 12, issued December 1990, pages 6121-6129, J A RAWLS et al., "Chemical Synthesis of Human Papillomavirus Type 16 E7 Oncoprotein: Autonomous Protein Domains for Induction of Cellular DNA Synthesis and for trans Activation", see whole document especially figure 1.
- Journal of Virology, Volume 65, No. 9, issued September 1991, pages 4681-4690, S A COMERFORD et al, "Identification of T and B-Cell Epitopes of the E7 Protein of Human Papillomavirus Type 16", see whole document, especially figure 1.
- Proceedings of the National Acamecy of Sciences (USA), Volume 88, No. 13, issued July 1991, pages 5887-5891, R W TINDLE et al, "A Public' T-Helper Epitope of the E7 Transforming Protein of Human Papillomavirus 16 Provides Cognate Help for Several E7 B-Cell Epitopes from Cervical Cancer-Associated Human Papillomavirus Genotypes", see whole document.
- Peptide Research 3 (1990) 162-166
- J Virology 63 (1989) 2885-2892
- Vaccine 8 (June 1990) 199

## Description

THIS INVENTION relates to a subunit papillomavirus vaccine which is protective against anogenital human papillomavirus (HPV) infection. The invention uses peptides which constitute an antigenic component of the vaccine.

It is well known (e.g. in "Papilloma Viruses and Human Cancer" edited by H. Pfister and published by CRC Press Inc. in 1990) that papilloma viruses can be classified into several distinct groups based on the host in which they infect. Human papilloma viruses (HPV) can be further differentiated into types 1-56 depending on DNA sequence homology. Types 16, 18 and 42 are associated with the majority of in situ and invasive carcinomas which may occur in the anogenital tract and in particular the cervix. In this regard, a number of cervical intra epithelial neoplasias and carcinomas of the cervix have been associated with HPV16 and HPV18. (Lancaster et al 1987: Cancer Metast. Rev. 6 653 and Pfister 1987. Adv. Cancer Res 48 113). These same two references also point out that papilloma viruses are small DNA viruses encoding up to 8 early and 2 late genes.

The protein from the expression of the early gene E7 varies between 93 to 127 amino acid residues. The E7 protein is the most abundant viral protein in HPV16 containing CaSki and SiHa squamous carcinoma cell lines and in HPV18 containing HeLa and C4-1 lines. (Seedorf et al. 1987. EMBO J. 6, 139). DNA transfection experiments implicate the E6 and E7 ORF proteins in in vitro transformation of mouse fibroblasts (Yasumoto et al. 1986. J. Virol, 57, 572), rat epithelial cells (Matlashewski et al. 1987. EMBO J.6, 1741) and primary human keratinocytes (Schlegel et al. 1988, EMBO J. 7, 3181; Pirisi et al. 1987. J. Virol. 61, 1061). Cooperation with an active ras oncogene leads to full transformation (Matlashewski et al. 1987. EMBO J. 6, 1741) and there is a requirement for continued expression of the E7 gene to maintain the transformed phenotype (Crook et al. 1989. EMBO J. 8, 513). The E7 protein may be recognised by the immune system, since anti E7 antibodies can be detected in the serum of approximately 20% of patients with HPV16 associated cervical lesions (Jenison et al. 1988. J. Virol. 62, 2115; Jochmus-Kudielka et al. 1989. J. Natl. Cancer Institute 81, 1698; Smillie et al. 1990. Immunol. Infect. Dis. 1, 13).

In addition to types 16, 18 and 42 further genotypes 6, 11, 31, 33, 35 and 39 which fall within the same sub group as types 16, 18 and 42 are infective for anogenital epithelium (Gissman Cancer Surveys 3. 162-181 (1984) Zur Hausen & Schneider The Papillomaviruses p245-263 Edited by Howley and Salzman New York Plenum Press (1987). The DNAs of HPV types 16 and 18 are frequently found in genital tumours Durst et al J. Gen Virol. 66 1515-1522 (1985), Gissman et al PNAS 80, 560-563 (1983) supporting the concept that members of this sub group have an essential role in the etiology of genital cancer (Syrjanen et al British Journal of Obstetrics and Gynaecology 92 1086-1092 (1985). Integration of HPV16 DNA into host genomes is frequently observed in cervical cancers with interruption of the E2 viral ORF, the protein product of which region transregulates early ORF transcription from the p97 promoter and retention of intact E6 and E7 ORFs.

Abundant circumstantial evidence implicates host immune mechanisms in the control of HPV associated tumours of the anogenital epithelium (Singer et al British Medical Journal 288, 735-736 1984). There is an increased incidence of pre-neoplastic (Frazer et al Lancet ii 657-660 1986) and neoplastic associated lesions in homosexual men immunosuppressed by human immunodeficiency virus infection and a markedly increased risk of squamous cell carcinoma (SCC) of the cervix and vulva but not of control organs such as breast and rectum in immunosuppressed allograft recipients (Sheil and Flavel Ninth Report of Australian and New Zealand Combined Dialysis and Transplant Registry pp 104-112 Edited by APS Disney 1986).

Taken with the above, the normal natural history of HPV infection in most patients with alpha-gamma globulinemia suggests that cellular rather than humoral responses are important for the control of the phenotypic expression of HPV infection (Kirschner Progress in Medical Virology 1986).

Standard immunological approaches to the study of anogenital HPV infection have been hampered by the lack of a suitable animal model and of an in vitro epithelial cell culture permissive for HPV.

Vaccines have also been proposed in regard to HPV with however only indifferent success.

It has been proposed to use vaccines containing autogenous tumor homogenates [Abcarian et al J. Surg Res 22: 231-236 (1977) Dis Colon Rectum 25:64851 1982 Dis Colon Rectum 19: 237-244 (1976)]. However it has recently been advocated that patients should no longer be treated with autogenous vaccines because of the potential oncogenic effect of the viral DNA (Bunney 1986 Br Med J 293 1045-1047).

In relation to production of genetically engineered vaccines this matter has been discussed in Pfister (1990) above and it seems that difficulty has been experienced in obtaining an effective vaccine because of the plethora of different papilloma virus types. Pfister however points out that attention should be directed to the so called early proteins (ie. E1, E2, E3, E4, E5, E6, E7 or E8) because these proteins are most likely synthesised in the proliferating basal cells of a wart infection in contrast to the structural proteins which are expressed in the upper epidermal layers. Therefore according to Pfister (1990) virus capsid protein appears to be limited in relation to use in a vaccine. The use of recombinant vaccinia viruses in in vitro test systems for papilloma virus early proteins in eukaryotic cells has been discussed also in Pfister (1990). This may take the form of a live vaccine consisting of genetically modified vaccinia virus expressing papilloma virus proteins or on the surface of paraformaldehyde fixed autologous cells infected in vitro with vaccinia recombinants or transfected with other expression vectors. Another strategy for vaccine development as discussed in Pfister (1990) is to use an immune stimulating complex of the glycoside QuilA.

Data on successful prophylactic vaccination exist only for bovine fibropapillomas homogenised homogenate of bovine fibropapillomas and has been shown to provide limited immunity [Olson et al J am Vet Med Assoc 135, 499 (1959) Cancer Res 22 463 (1962)]. A vaccine including an engineered L1 fusion protein (Pilacinski et al. UCLA Symp. Molecular and Cellular Biology New Series Vol 32 Papilloma Viruses Molecular and Clinical Aspects Alan R Liss New York 1985 257) has also been used in calves but proved unsuccessful in humans. In Pfister (1990) it is stated that there is presently no evidence for a possible prevention of HPV infection by the use of a capsid protein vaccine, but induction of an antitumor cell immunity appears to be feasible.

The L1 and L2 genes have been the basis of vaccines for the prevention and treatment of papilloma virus infections and immunogens used in the diagnosis and detection of papilloma viruses (International Patent Specifications WO8605816 and WO8303623). However, it appears that no commercial usage of these vaccines have taken place.

Reference may also be made to Patent Specification EP386734 which describes new immunogenic regions of HPV16 E7 protein which may be useful in vaccines, EP 375555 which describes HPV16 peptides useful as immunoassay reagents for the detection of HPV16 proteins and which contain an antigenic determinant for HPV16, a reference in VACCINE (1990) 8 3, 199-204 which describes vaccines including recombinants expressing HPVE5, E6 or E7 ORF intended for use in providing antitumor activity, Australian Specification 52860/90 which describes screening antibodies for specificity to an antigen which is an epitope of HPV16 L1 or E7 proteins, AU-A-75535/87 which describes synthetic peptides of HPV corresponding to an amino acid sequence region having at least one reverse turn and predicted hydrophilicity, EP217919 which describes type specific papillomavirus DNA sequences and peptides useful in vaccines containing 15-75 nucleotides, US-A-4551270 which describes at least one antigenic determinant of papillomavirus and immunogens and vaccines containing the antigenic determinant, EP412762 which describes a polypeptide having the sequence Leu-Tyr-Cys-Tyr-Glu-Gln-Leu--Asn-Asp-Ser-Ser which inhibits binding of the HPV E7 protein to retinoblastoma gene which may be used in vaccines for treatment of cervical cancer and genital warts. FR-A-2643817 which describes a vaccine for treatment of tumours induced by papillomavirus containing recombinant poxvirus with heterologous DNA encoding region of non structural papillomavirus, JP-A-O1061665 which describes an antibody formed to an antigen polypeptide of HPV16 E6 or E7 protein which antigen polypeptide is Tyr-Gln-Asp-Pro-Gln-Glu-Arg-Pro-Arg-Lys-Leu-Pro-Gln-Leu-Cys which is part of E6 protein or Cys-Tyr-Gln-Leu-Asn-Asp-Ser-Ser-Glu-Glu-Asp-Glu-lle-Asp which is part of E7 protein, AU-A-76018/87 which describe expression products of HPV16 or HPV18 which may be used for the production of antibodies EP235187 which describes kits containing polypeptide(s) expressed by several groups of papilloma virus including HPV16 and HPV18 which are expression products of E6, E7 or L2 genes and US-A-4777239 which include diagnostic synthetic peptides for HPV one which includes residues 45-58 of protein E6 and 40-50 or protein E7 which may be used as therapeutic agents.

Of particular interest in the prior art discussed above is EP375555 which describes a peptide AEPDRAHYNIVTFC which may be used as an immunoassay reagent for diagnosis of HPV16 antibodies. This peptide includes the DRAHYNI sequence. However it is clear from a review of this document that there was no realisation that the DRAHYNI sequence corresponded to a T helper cell epitope of the ORF of E7 protein of HPV16 and the consequences in regard to HPV therapy as discussed in this patent specification.

Of particular relevance also is EP 386734 which discloses a number of peptides one of which (ie No. (V) comprises the sequence Asp-Glu-Ile-Asp-Gly-Pro-Ala-Gly-Gln-Ala-Glu-Pro-Aspm-Arg-Ala-His-Tyr). It will be noted that this sequence includes the sequence DRAHY. While this particular peptide is described as corresponding to a useful immunogenic region of HPV16 E7 protein, and thus useful in vaccines it will be appreciated from the discussions hereinafter that the sequence DRAHYNI has a more useful antigenic property and thus will stimulate a far greater immune response.

EP 451 550, which is effective under Article 54(3) EPC, discloses seroreactive epitopes of HPV 16 proteins E4, E6, E7. One of the amino acid sequences used in a vaccine is GPAGQAEPDRAHYNI.

In this specification amino acids are represented by single letter codes as follows:

| | | | |
|---|---|---|---|
| Phe: F | Leu: L | Ile: I | Met: M |
| Val: V | Ser: S | Pro: P | Thr: T |
| Ala: A | Tyr: Y | His: H | Gln: Q |
| Asn: N | Lys: K | Asp: D | Glu: E |
| Cys: C | Trp: W | Arg: R | Gly: G |

It is therefore an object of the invention to provide a subunit vaccine which may be utilised to treat HPV infections and which also may be used to provide immunity against HPV infection.

The peptides used in the invention have the structure DRAHYNI and structural homologues thereof which homologues concern a single amino acid substitution which peptide is linked directly or indirectly to one or more amino acid sequences which correspond to a B epitope of HPV16 or HPV18.

Suitable B epitopes may be selected from HPV E7 16 epitopes which include QAEPD, IDGP, EYMLD and YMLD. Suitable B epitopes that may be selected from HPV E7 18 epitopes include DEIDGVNHQL.

Representative peptides which fall with the scope of the invention include the following:

| | |
|---|---|
| B1 - A1 - DRAHYNI - A2 | (1) |
| B2 - DRAHYNI - B3 | (2) |
| B1 - A1- DRAHYNI - A2 - B4 | (3) |
| B2 - DRAHYNI - A2 | (4) |
| A1 - DRAHYNI - B3 | (5) |
| A1 - DRAHYNI - A2 - B4 | (6) |
| B1 - A1 - B4 - A1 - DRAHYNI - A2 | (7) |
| B1 - A1 - B4 - DRAHYNI - B3 - A2 - B5 | (8) |
| B1 - A1 - B4 - A2 - B2 - DRAHYNI - A2 | (9) |

In the above formulae (1) through (9) B1, B4 and B5 represent B epitope sequences that may be linked to the T epitope sequence indirectly through intervening sequences of amino acids that are not B epitope sequences such as A1 and A2. The peptides do not include those containing GPAGQAEPDRAHYNI.

In some cases the B epitope sequence may be linked directly to the T helper epitope sequence and in such a case in a first situation the terminal amino acid of the B helper epitope sequence and the first amino acid of the T epitope sequence may be merged. In other cases in a second situation the last amino acid of the T helper epitope sequence and the first amino acid of the B epitope sequence may also be merged. In this embodiment therefore B1 represents a B epitope sequence which refers to the first arrangement and B3 represents a B epitope sequence that represents the second arrangement. Examples of the first and second situations are

| | |
|---|---|
| QAEPDRAHYNI - A2 | (10) and |
| A1 - DRAHYNIDGP | (11) |

In formulae (10) and (11) the amino acid D which corresponds to aspartic acid represents the first situation and in formula (11) the amino acid I which corresponds to isoleucine represents the second situation.

An especially preferred peptide that may be used in the invention is the peptide QAEPDRAHYNI - A2

The sequence DRAHYNI in accordance with the present invention has been identified as corresponding to a major T helper cell epitope in the E7 open reading frame (ORF) of HPV16 and HPV18. DRAHYNI corresponds to amino acids 48-54 of the E7 ORF.

The abovementioned peptides used in the invention which may be formed synthetically may form immunogens capable of eliciting strong antibody response to HPV16 or HPV18 E7 challenge. The T epitope may facilitate the production of antibody to several B epitopes simultaneously.

The invention includes within its scope a vaccine which includes one or more of the abovementioned peptides in combination with a suitable adjuvant. For animals infected with HPV suitable adjuvants may be selected from Freunds Complete Adjuvant, Freunds Incomplete Adjuvant, QuilA and saponins generally. In relation to humans it is preferred to utilise an adjuvant which is compatible with humans and an appropriate adjuvant in this regard is ISCOMS (ie immuno stimulating complexes).

The invention is significant because of its therapeutic value in relation to cervical cancer which like AIDS has a devastating effect on human lives. If a vaccine against these diseases could be developed, it is to the greatest benefit of the mankind. Immunisation with attenuated or killed viruses may have inherent risks associated with it. To reduce the risk one could use small peptides containing the necessary B and T epitopes as vaccines. The major advantages of peptide vaccines are (i) feasibility to obtain large quantities of relatively pure peptides by automated chemical synthesis, (ii) the ability to tailor the peptide in such a way that the useful B epitopes could be incorporated in to the vaccine construct while deleterious B epitopes could be left out, (iii) If necessary many artificial T-helper epitopes could be incorporated in a mixed vaccine to help overcome MHC restriction in an out bred population.

It is easy to induce antibodies against peptides using Freund's adjuvant in animals provided the peptide concerned has a T-helper incorporated into its sequence. However one has to be able to use an adjuvant less harmful than Freund's, but with equal efficacy when used on humans. Also ideally the adjuvant should be able to elicit CD8+ MHC class I restricted cytotoxic T lymphocytes, when using peptides as immunogens. Immunostimulating complexes (ISCOMS) have the potential of satisfying both the above conditions ISCOMS are stable molecular structures, with a mean diameter of 35nm, in which protein antigens are incorporated into a matrix of cholesterol and an adjuvant glycoside QuilA.

In order to incorporate a protein into ISCOMS the protein has to have a lipid binding region. Recently non lipid binding proteins were bound to ISCOMS by exposing hidden lipid binding regions of the proteins by changing the pH or by coupling with a protein known to bind lipids. In these studies the researchers have used the whole length proteins which may not be safe to use on humans and/or which are hard to prepare to purity necessary for human use. In this specification a method is described hereinafter of preparing vaccines using small synthetic peptides of defined B and T cell epitopes coupled to a synthetic lipid binding peptide, in mice. Since many peptides do not bind lipids on their own they could be made lipid binding by coupling to the lipid binding peptide LAP20 which takes an amphipathic α-helical confirmation in the presence of lipid.

### MATERIALS AND METHODS

### Synthetic Peptides.

Peptides were synthesised using the simultaneous multiple peptide technique originally described by Houghten (PNAS USA 82 5131-5135 1985) employing derivatised t-Boc amino acids on benzhydryl resin, or using Fmoc chemistry on an Applied Biosystems 431 A Peptide Synthesiser. Peptides were routinely analysed for homogeneity by HPLC. Peptides less than 90% pure were purified. The amino acid composition of all peptides was checked, and peptide 8Q was amino acid sequenced. Data were confirmed using 2 or 3 separate syntheses of peptides and two different chemistries (Fmoc and t-Boc) to preclude batch idiosyncrasy. All peptides were tested for non-specific mitogenicity on unprimed lymph node cells and for toxicity on tuberculin PPD- primed T-cells. Stock solutions were made by dissolving peptides in tissue culture medium at 5 mg. per ml. In some cases acetic acid to 5% was added to attain solution.

### HPV16 E7 protein.

HPV16 E7 protein was produced as MS2 fusion protein (FP) from a heat inducible phage promoter in a pPLc 24 expression vector (provided by L. Gissmann) in E.coli 600/537.FP was partly purified from lysozyme-disrupted bacteria by Triton-X 100 and sequential urea extraction as described (Seedorf et al 1987 EMBO J. 6, 139-144). Purification was monitored on PHAST (Pharmacia) SDS-PAGE. Preparations containing 60-90% pure FP as judged by appropriately sized major bands on gels, were obtained as 8-10 M urea extracts.

### Lymph node cell (LNC) Proliferation assays.

Mice were immunised subcutaneously in the base of the tail with 20-50 ug. of peptide emulsified in complete Freunds adjuvant (H37 Ra.CFA Difco Labs. Detroit). Eight to ten days later mice were killed, ingurinal and periaortic nodes were removed and a suspension of lymph node cells prepared. Cells were plated in triplicate at 4x10⁵/0.2 ml in flat bottomed 96 well microtitre plates in Hepes buffered RPMI 1640 medium containing glutamine, pyruvate, 2% heat inactivated mouse serum and 5x10⁻⁵ M 2-mercaptoethanol, and antigen at various concentrations. After 4 days, cells were pulsed with luCi of (³H)-thymidine (5 Ci/mmol, Amersham, U.K.) and after a further 18 hours, incorporated ³H was quantified by B-emission spectroscopy.

### Mice.

In-bred mouse strains were obtained from University of Queensland animal breeding facility or from animal Resources Inc. Perth, Western Australia. Mice were used at 8-24 weeks old.

### Assay for cytokine production.

The lymphokine (interleukin-2 (IL-2) and interleukin-4) dependent HT-2 cell line was maintained in vitro in RPMI medium supplemented with 10% fetal bovine serum and 10% supernatant from the IL-2 producing cell line MLA 144. Omission of MLA supernatant resulted in cessation of cell division within 18 hours, as measured by (³H)-thymidine incorporation. Supernatants from LNC proliferation assays were harvested at 3 days and tested for induction of proliferation of HT-2 cells (Ertl et al (1989) J. Virol. 63, 2885-2892) which had been starved of MLA supernatant overnight and washed extensively in serum free medium. 2x10³ HT-2 cells in 100 ul RPMI medium supplemented with 10% FCS were cultured in triplicate with 50ul of LNC proliferation assay supernatant, which had been centrifuged to move residual lymph node cells. Proliferation was measured 40-48 hours later by a 6 hour (³H)-thymidine pulse (0.5 uCi per well).

### Peptide Elisa assay.

Peptides 3Q, 6Q, 7Q (not claimed) and 8Q were conjugated to bovine serum albumin (BSA) using a single step glutaraldehyde method as described (Avrameus 1969-Immuno Chemistry 6, 43-47). Peptide-BSA conjugates were bound to microtitre plates by incubation at 50ug/ml in bicarbonate binding buffer pH 9.6. Remaining binding sites on the microtitre plates were blocked with phosphate buffered saline (PBS) containing 5% BSA prior to incubation with serum from mice immunised with various peptide constructs, at a range of dilutions in PBS containing 5% non-fat milk powder, 0.1% BSA, 0.1% Tween-20. After appropriate washings, horseradish peroxidase conjugated anti-mouse Ig. (Silenus Laboratories, Australia) and 2,2¹-azinobis (3-ethyl-benzthiazoline sulfonate) (ABTS) substrate were added. Optical density (O.D.) was quantified on a Titertek multiscan microtitre plate reader (Flow Laboratories, Scotland) at 414 nm. HPV16 E7/MS FP Elisa assays were conducted with modifications as described (Tindle et al 1990 J. Gen Virol 71, 1347-1354). All ELISA assay plates contained wells which were concurrently incubated with a panel of monoclonal antibodies 8F, 4F and 6D, specific for HPV16 E7 linear epitopes EYMLD, IDGP and QAEPD respectively (Tindle et al (1990) Peptide Res. 3, 162-166), as positive and negative controls.

### Peptide Immunisation for antibody production.

Mice were immunised 3 times intraperitoneally (ip.) with 20-50 ug of peptide emulsified in complete Freund's adjuvant at 14 day intervals. Mice were bled from the retro-orbital plexus 8 days after the last injection, serum prepared, and ELISA was performed.

### Carrier Priming Assay.

Mice (3-5 per group) were immunised ip. with 20-50 ug. of peptide 8Q or PBS emulsified in CFA. Three to five weeks later, mice were infected by tail base scarifaction with 10⁷ plaque forming units (pfu) of recombinant vaccinia virus containing the entire HPV16 E7 gene (VAC-E7) (Drs. A. Minson and J. Sterling, pers. cons. ) or 10⁷ pfu of wild-type vaccinia virus (WR-VAC). 7-8 days later, serum was prepared from each mouse, and anti-E7 antibodies were determined by ELISA assay against peptide 8Q or HPV16 E7 FP bound to microtitre plates. Negative controls were mice immunised with irrelevant peptide (6Q), and microtitre plates to which irrelevant peptides were bound.

### RESULTS

### Lymph Node Cell Proliferation Assays.

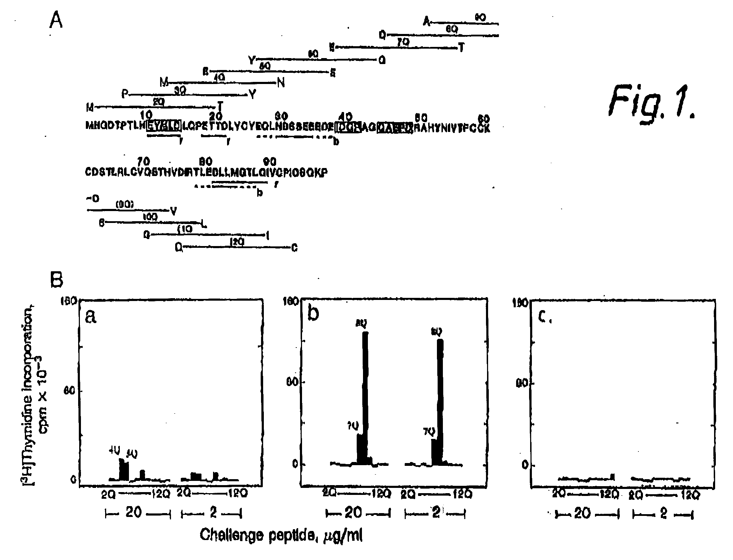

A set of overlapping 15-20 mer peptides covering the entire predicted HPV16 E7 protein (Figure 1A) was used to located T-proliferative epitopes. 4 groups of C57B1/6(H-2^{b}) mice were immunised with mixtures of peptides 2Q-5Q, 6Q-9Q or 10Q-12Q in CFA, or with RPM1 in CFA. Pooled LNC from each group were challenged in vitro with 2 or 20 ug/ml of individual peptides and proliferation measured as incorporation of radiolabelled thymidine. The data shown in Figure 1B are representative of 6 assays. Peptide 8Q consistently elicited strong proliferation in LNC from the 6Q-9Q immunised group (Figure 1B.b). Peptide 7Q elicited a weaker response in this group. Peptides 8Q and 7 Q share a 12 amino acid overlap at position 44-55. Weak and inconsistant responses were seen in LNC from 2Q-5Q immunised mice, when challenged with peptides 4Q and 5Q (3 out of 6 experiments) Figure 1B,a). No further peptides from the 2Q-12Q series induced proliferation in assays using LNC from appropriately immunised B10.A(4R)(H-S^{h2}), or Balb/c (h-2^{d}) mice (data not shown).

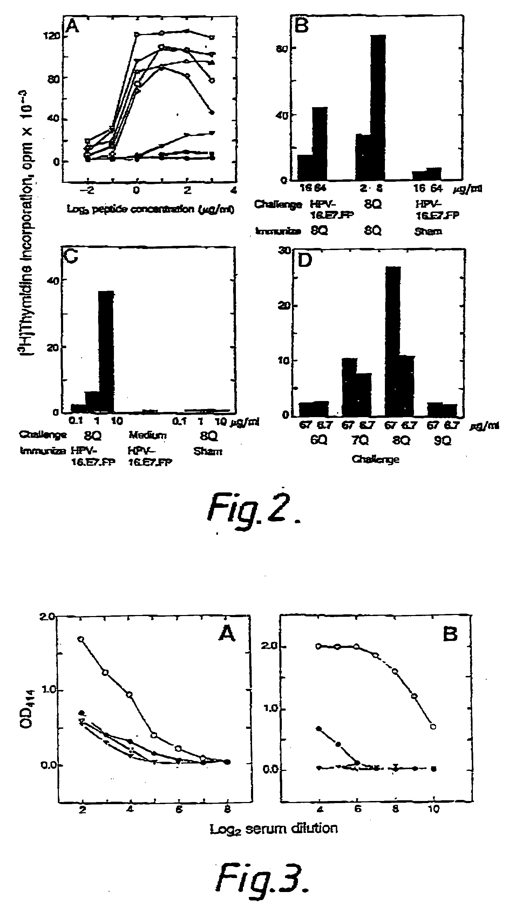

In order to investigate the MHC restriction of the proliferative response to 8Q, a series of congenic mice, differing only at the MHC class 2 locus, and other in-bred strains of MHC class 2 haplotype-defined mice, were immunised with mixtures of peptides 8Q and 6Q and their LNC subsequently challenged in vitro with 8Q or 6Q (6Q was included as an internal negative control). Immunised mice from all congenic strains on a B10 background showed strong proliferative responses to peptide 8Q, but not to control peptide 6Q, over 0.04-27 ug/ml range (Figure 2a). In further experiments other immunised strains S7R(I-A^{s}I-E^{s}), S94(I-A^{s}I-E^{kBS}), C₃H(I-A^{k}I-E^{k}), CBA(I-A^{k}I-E^{k}), DBA(I-A^{d}I-E^{d}), Balb/c (I-A^{d}I-E^{d}), C57B1/6(I-A^{b}I-E^{b}) and BL10(I-A^{b}I-E^{b}) all showed proliferative responses to 8Q. These data indicate that the proliferative response to peptide 8Q in previously primed mice is not restricted through any of the 5 I-A or 5 I-E alleles tested.

In order to define the minimal peptide which would induce proliferation, LNC from 8Q immunised mice were challenged in vitro with a series of C'-terminal and N'-terminal truncations of 8Q (Table 1). LNC stimulated with peptides B3, B4, 8Q and B7-10 inclusive showed significant proliferation, indicating that the consensus sequence ⁴⁸DRAHYNI⁵⁴ was the minimal proliferative epitope. In a subsequent experiment, LNC from 8Q primed B10.A(2R) and 29R mice proliferated in response to the 7-mer peptide DRAHYNI though the stimulation indices were much lower (6.1 and 5.1 respectively).

The ability of peptide 8Q to prime for a response of LNC to in vitro challenge with HPV16 E7 protein was tested. The proliferation elicited by challenge of LNC from peptide 8Q immunised mice with HPV16 E7 FP and 8Q was of the same order of magnitude, provided challenges were adjusted to be approximately equimolar for 8Q (Figure 2B).

In an experiment to test whether HPV16 E7 protein would prime for peptide 8Q, LNC from mice immunised with HPV16 E7, but not 'sham' immunised mice proliferated when challenged in vitro with 8Q (Figure 2C).

### LNC from primed mice produce interleukins when stimulated in vitro.

Supernatant fluid from 8Q and 7Q (not claimed) challenged, but not 6Q or 9Q challenged, LNC from mice previously immunised with a mixture of 6Q-9Q peptides, induced proliferation of the IL-2/IL-4 dependent cell-line HT-2, (Figure 2D). Supernatants of LNC from mice immunised, and challenged in vitro, with the other Q-series peptides failed to induce proliferation of HT-2 cells (data not shown).

Having determined that peptide 8Q contained a T-epitope to which primed LNC would respond by proliferation and cytokine production, we then undertook a series of experiments to determine whether primed LNC would provide 'help' to B cells for the production of specific antibody to B cell epitopes of HPV16 E7. Earlier work from our laboratory has defined the location of 3 immunodominant B-cell linear epitopes in the HPV16 E7 protein, recognised by murine monoclonal antibodies (Mabs) (Tindle et al 1990. J. Gen. Virol. 71, 1347-1354). In initial experiments we exploited the fact that peptide 8Q, in addition to the T-epitope ⁴⁸DRAHYNI⁵⁴ defined above, also contained an immunodominant B-epitope, ⁴⁴QAEPD⁴⁸.

### Mice Immunised with 8Q peptide respond to in vivo challenge with recombinant vaccinia virus containing the HPV16 E7 ORF gene by production of antibody to the E7 protein.

The sera of mice immunised with 8Q and infected 3½ weeks later with recombinant vaccinia-E7 virus (VAC-E7), but not wild-type virus (WR-VAC), contained antibodies reactive with 8Q (Figure 3B) and with HPV16 E7 (Figure 3A), both of which contains the QAEPD B-epitope.

It is therefore clear that a single immunisation with 8Q peptide primed DRAHYNI-reactive T-helper (Th) cells and also B-cells which recognise the QAEPD-containing peptide for subsequent challenge with whole eukaryotic E7 protein is processed in such a way as to stimulate primed DRAHYNI-reactive Th-cells to provide help for B-cells producting antibody to the QAEPD-containing peptide.

### Mice immunised with peptides containing the T-epitope DRAHYNI and B-epitope (s) of HPV16 E7 or HPV18 E7 produce antibodies which specifically recognise E7 protein.

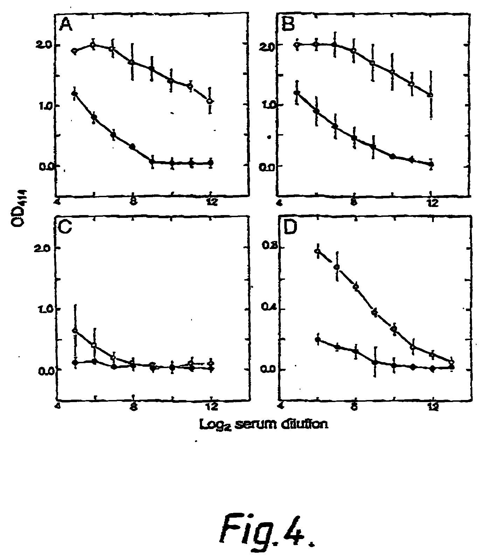

Sera from mice immunised with peptide 8Q reacted in ELISA assay with 8Q and 7 Q (not claimed) (Figure 4A, B) and HPV16 E7 FP (Figure 4D) but not control peptides 6Q (Figure 4C), or 2Q, 4Q and 10Q (data not shown). These data suggested that the serum antibodies may have recognised the B-epitope QAEPD contained within 8Q, 7Q and HPV16 E7. In a further series of experiments, the sera from some mice immunised with peptides B7 or B8 reacted with 8Q and 7Q, whereas sera from mice immunised with B16, B17, B19 or B3 did not (Table 2). The data indicated that in order to elicit an antibody response to peptides and HPV16 E7 FP containing the B-epitope QAEPD, the immunogen was required to contain the T-epitope DRAHYNI in addition to the sequence QAEPD.

Further experiments were carried out to determine if the inclusion of T-epitope DRAHYNI in synthetic peptides used for immunisation would result in help being extended to sequences containing B-epitopes other than QAEPD. The sera of 3 mice immunised with peptide B11 all contained antibodies to B-epitopes EYMLD and QAEPD, and reacted with HPV16 E7 (Table 2). Reactivity with HPV16 E7 could be absorbed out by pre-incubation of the sera with peptide 8Q (containing QAEPD) and 2Q (containing EYMLD). The serum of one of these mice also contained antibodies to B-epitope IDGP. The sera of mice immunised with 7Q (not claimed) contained antibody to QAEPD, but not IDGP (Table 2).

In order to determine if the inclusion of DRAHYNI in a synthetic peptide containing a B-epitope from the putative E7 protein of an HPV genotype other than HPV16 could drive the production of heterologous antibody, mice were immunised with peptides GF11, GF12 and GF15 containing an immunodominant linear B-epitope DEIDGVNHQHL of HPV18 E7 (Selvey et al 1990. J. Immunol. 145, 3105-3110). Serum antibodies which recognised a peptide (GF13) containing the B-epitope, and whole HPV18 E7 were producded in all 3 mice immunised with GF15, which contains intact DRAHYNI, but not in mice immunised with GF12 or GF11 where the T-epitope is truncated or absent. GF15 immunised mice simultaneously produced antibody which recognised the HPV16 E7 B-epitope QAEPD and the whole HPV 16 E7 (Table 2).

In mice immunised with synthetic peptides containing both T- and B-epitopes, and which produced antibody, presumably both sets of lymphocytes ie. The cells and antibody producing B-cells were primed.

### Immunisation with T-epitope alone can prime for an antibody response.

Mice immunised with peptide B3 (containing T-epitope DRAHYNI but no B epitope) and challenged with peptide B7 (containing B-epitope QAEPD and T-epitope DRAHYNI) produced antibody detectable at 5 days which recognised 8Q and whole HPV16 E7 FP (Table 2). Mice challenged with B3 did not produce antibody. Nor did mice which had been immunised once.

### Day zero proliferative assay

PBMC were separated from 60ml of blood on Ficoll Hypaque and washed three times in RPM1 1640. The cells were courted and diluted to give 10⁶ cells/ml ie. 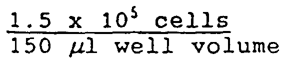 in complete RPM1 1640 with 10% human pool AB. In this experiment 150 µl of cells at 10⁶ cells/ml were added to the wells of a 96 well U bottomed plate as set out in Table 4. Cells were challenged in triplicate with peptides 101-109 identified in Table 3. (Note that peptide 106 corresponds to peptide 8Q). From Table 4 it will be noted that the cells were challenged with 106 alone and other peptides in groups of three at two concentrations with and without PHA. Tetanus toxoid and media alone wells were included as positive and negative controls respectively. The cells were then incubated 7 days at 37°C with 5% CO₂ and labelled overnight with 1 µc/ml ³H thymidine. The cells were then harvested, dried and counted and results recorded as counts/minute. The procedure was also modified to challenge cells in quadruplicate and at a peptide concentration of 5 and 15 µg/ml. The results are recorded in Table 5.

### Binding of peptides to ISCOMS

Peptides were synthesized using Fmoc chemistry on an Applied Biosystems 431A peptide synthesiser. Purity of the peptides were checked by HPLC and amino acid analysis and found to be of greater than 90%.

Peptides synthesised:BT5 (A peptide from HPV16 E7 containing the B epitope QAEPD and the T-helper epitope DRAHYNIVTFCCKCD), QAEPDRAHYNIVTFCCKCD. LAP20 **(The synthetic lipid binding peptide),** VSSLLSSLKEYWSSLKESFS. 7Q (A peptide from HPV16 E7 containing the B epitope QAEPD), EIDGPAGQAEPDRAHYNI, both not claimed. GF110 (A peptide from HIV virus coupled to the HPV16 E7 T-helper epitope DRAHYNIVTFCCKCD), TRKSIRIQRGPDRAHYNIVTFCCKCD

### Preparation of ISCOMS

The ISCOMS were prepared by the dialysis procedure wherein briefly 2mg phosphatidyl choline and 1 mg cholesterol was dissolved in a few drops of chloroform and the solvent removed under a stream of nitrogen. The dried lipid mixture was dissolved in 3 ml Tris buffer (20mM Tris/HCl pH=7.8, 150mM NaCl containing 1% octylglucoside. 2mg peptides in 2ml PBS and 4 ml of a 10% quil-A solution in water was added to the lipid mixture and mixed for 1 hour at room temperature. The mixture was extensively dialysed against PBS overnight at 4°C. The precipitate formed during dialysis was removed by low speed centrifugation. The supernatant was collected and the ISCOMS were separated from free peptide and Quil-A by layering the supernatant on a 10% sucrose in PBS cusion and allowing the ISCOMS to pellet through by centrifugation using a Beckmann TLA 100.3 rotor at 40 000 rpm for 16 hours at 6°C. The ISCOM pellet was dissolved in PBS.

CBA mice 6 to 10 weeks old were used in immunisations. Mice were injected subcutaneously at the base of the tail with 20ug peptides coupled to ISCOMS, and as a positive control 100ug peptide in Freund's complete adjuvant. As a negative control 20ug of BT5/LAP20 in PBS or BT5 with Quil-A and/or lipid was used. After 21 days the mice were bled and boosted with another injection, and after a further 14 days they were bled again. The antibodies were detected by ELISA plates prepared with peptides themselves or recombinant proteins derived from MS2 fusion proteins from E. coli or recombinant baculovirus infected Spodoptera cells.

### Monitoring of peptide binding to ISCOMS

At each stage of the preparation of ISCOMS, protein assays were performed using BCA protein assay kit (Pierce Chemical Co), and bovine serum albumin was used as the protein standard.

### Discussion

In the present study we define a major proliferative T-epitope ⁴⁸DRAHYNI⁵⁴ in HPV16 E7, which stimulates the T-cells of all strains of mice of defined haplotype which we have tested. DRAHYNI stimulates cytokine production in responding T cells, and when coupled to a homologous B-epitope and injected into mice, can elicit cognate help for the production of specific antibody which recognises native E7 protein.

As the 2 overlapping peptides 7Q (not claimed) and 8Q stimulated proliferation in primed T cells (Figure 1), sequences common to both are likely to be responsible. Proliferation experiments with C- and N-terminal truncations of peptide 8Q indicated that the sequence DRAHYNI was the minimal reactive epitope. Our data do not completely exclude the possibility that more than one distinct T cell site may be responsible for proliferation mediated by 7Q and 8Q but we consider it unlikely, since the 7 N-terminal amino acids of 7Q not shared with 8Q are all contained, within peptide 6Q which does not contain a proliferative epitope. In many repeated experiments, the proliferative response to 8Q was always an order of magnitude greater than that to 7Q in LNC from appropriately primed mice, suggesting that flanking sequences outside the minimal epitope DRAHYNI could influence response (Rothbard et al 1988 EMBO Journal 7, 93-100). Further evidence for this supposition was the observation that while challenge of LNC from 8Q primed mice with the 7-mer DRAHYNI induced proliferation, the magnitude of the response was higher when DRAHYNI was presented with C- or N-terminal elongations (Table 1, and text). In some experiments LNC from mice which had not been specifically primed showed low levels of proliferation in response to co-culture with 8Q peptide (Figure 2A legend). This may be a primary response in vitro and would suggest that the frequency of T-precursors whose TcR recognises DRAHYNI may be high.

It is perhaps surprising that only one major T epitope was identified in the entire HPV16 E7 molecule using the Q-series range of peptides and the 5 MHC haplotypes were used. Minor proliferation induced by 4Q and 5Q both of which contain algorithm predicted T-sites (Figure 1) was observed in primed B10A(2R) and C57B1/6 mice although no cytokine production was detectable. The putative T-sequence of 4Q was unable to provide help for antibody production to the adjacent EYMLD B-epitope when peptide 3Q, which contains both, was used to immunise mice. Nor could 4Q prime mice for antibody production upon subsequent in vivo challenge with VAC-E7. Although our panel of overlapping peptides was designed to cover the E7 molecule comprehensively, we cannot be entirely certain that other T-cell sites have not been missed, particularly if residues distant from a putative epitopic site can influence T-epitope recognition. DRAHYNI was not predicted by the DeLisi & Berzofsky (1985 PNAS USA 82, 7048-7072) or Rothbard (above) T-epitope algorithms. The predicted secondary structure of DRAHYNI is a turn or coil at its N-terminal end, while tyrosine and asparagine are likely to form part of a bata-strand extending C' terminally.

We have demonstrated clearly that immunisation of mice with DRAHYNI joined to a B-epitope will elicit antibody reacting specifically with peptides containing the B-epitope and with the whole E7 molecule.

Furthermore, immunising mice with a peptide containing DRAHYNI but no B-epitope elicited a secondary antibody response when mice were subsequently challenged with DRAHYNI plus B-epitope, suggesting that T-activation in the absence of a B cell response is sufficient to prime.

For developing a peptide vaccine, the candidate should have the capability to elicit in vivo a T cell response to the whole native molecule from which the peptide derives. We have shown that a single priming shot of a peptide containing DRAHYNI and the QAEPD B-epitope will induce immunological memory which can be recalled by in vivo subsequent infection with live vaccinia-E7 recombinant virus containing the full length E7 gene. This latter observation indicates that eukaryotic whole E7 may be processed and presented to the immune system in a way that can be seen by antibody secreting B-cells whose functional development has depended on cognate help provided by Th cells stimulated by DRAHYNI. The relevance of the anti-peptide response to the recognition of whole eukaryotic E7 protein is further indicated by the observation that murine anti-QAEPD monoclonal antibody recognises native HPV16 E7 in CaSki cells in immunoprecipitation (Tindle et al 1990. Peptide Res. 3, 162-166). In other virus infections the relevance of defined Th and Tc epitopes for anti-viral protection in vivo has been documented for viral proteins which like E7 are not expressed on cell surfaces, eg influenza A virus nucleoprotein (Townsend et al 1986 Cell 44, 959-968), cytomegalovirus immediate early protein p89 (del Val et al J. Virol 62, 3965-3972 1988), as well as those on the cell surface which probably protect by inducing neutralising antibodies.

The experiments in which mice were immunised with DRAHYNI and B-epitopes linked in various conformations indicated that DRAHYNI could provide cognate help to more than one clone of antibody secreting B cells to produce multiple antibodies of different specificities. It was not the purpose of these experiments to test all permutations, but it was clear that the production of antibody occurred in several combinations of the position and orientation of the B-epitopes with respect to the T-epitope. Similar findings on epitope orientation have been reported by others (Leverly et al Cell Immunol 125, 65-78 1990) (Good et al 1987 Science 235, 1059 - 1062). We have no data to indicate whether DRAHYNI is the T-epitope responsible for providing help for B cells producing anti-QAEPD, anti-EYMLD and anti-IGPD antibody in mice immunised with whole E7/MS2 FP (Tindle et al 1990 Peptide Res. 3, 162-166) where B- and T-epitopes are in their natural configurations as it does in experiments reported here in which the B- and T-epitopes are closely linked. In this context it has been reported that immunodominant Th sites are frequently near the B cell site (Manca et al 1985 Eur. J. Immunol. 15, 345-350).

DRAHYNI fulfils the criteria of an effective T-epitope vaccine for use in out-bred populations, of being recognised in association with many different MHC haplotypes. It joins a small number of stimulating peptides recently described which are recognised in association with multiple MHC haplotypes (sinigaglia et al 1988) (Milich et al 1988 Proc. Natl. Acad. Sci. USA 85, 1610-1614), (Nicholas et al 1988 J. Virol. 62, 4465-4473), (Herber-Katz et al 1988 J. Exp. med. 167, 275-287), (Lai et al 1987 J. Immunol. 139, 3973-3980). While it is believed that requirements for binding to MHC are less stringent than those for binding to TCT (Sette 1987) it is nonetheless surprising that DRAHYNI caused proliferation in, and therefore presumably bound to, all tested Ia haplotypes. It has been suggested that widely reactive peptides are capable of forming a structure closer to an 'ideal' T-epitope that can associate with many class Ia alleles (Shrier et al 1989 J. Immunol. 142, 1166-1176). While introduction of strong heterologous T-epitopes into vaccines has been advocated (eg hepatitis B virus core antigen, (Stahl and Murray 1989 Proc. Natl. Acad. Sci. USA 86, 6283-6287)) ideally synthetic HPV vaccines would be composed of T and B cells sites derived from the same organism so that latent and/or subsequent infections would elicit a response from both populations of lymphocytes. Such natural boosting is important if constant high levels of antibody are required for protection. A response to an HPV encoded T-epitope is also critical if antibody independent T-cell immunity is required for protection.

Immunisation of mice with whole HPV16 E7 produced as a recombinant fusion protein with MS2 replicase in bacteria, primed for subsequent in vitro challenge with 8Q. The magnitude of the response, however, was consistently lower and less reproducible than priming with peptide. Why whole E7 protein primes for 8Q peptide less well than 8Q primes for 8Q was not addressed in this study but presumably relates to processing and presentation of fragments of the priming antigen to the immune system.

A vaccine for prophylactic and therapeutic use to eradicate HPV infection is desirable, since destructive elimination of all virus infection does not appear technically feasible and no specific anti-viral agent is available. 'Attenuated' HPV alone is unlikely to prove sucessful as a potential vaccine because of its extremely restricted host cell range and its constitutive lack of infectivity. Furthermore, the use of live HPV to vaccinate is out of the question because of the inherent risk that putative HPV oncogenes will integrate into host cell DNA. In any case, this approach is precluded since there is no tissue culture or animal system for producing large amounts of whole HPV. The development of a peptide vaccine requires the delineation of B- and T-epitopes within ORF peptides recognised by the host's immune system, and the interaction between the epitope responsive cells resulting in specific antibody and cytotoxic effectors. Our group has recently defined immunodominant B-epitopes in HPV 16 E7 and HPV18 E7 peptides (Tindle et al 1990 J. Gen. Virol. 71, 1347-1354, Tindle et al 1990 Peptide Res. 3, 162-166, Selvey et al 1990 J. Immunol. 145, 3105-3110).

T-epitope DRAHYNI was identified initially by priming mice in vivo with mixtures of overlapping 11-20 mer peptides spanning the entire putative HPV16 E7 protein as translated from DNA (Seedorf et al 1985) and challenging cells from draining lymph nodes in vitro with individual peptides. Studies with other viruses have shown convincingly that T-epitopes relevant to infection with native virus can be defined by synthetic peptides using a similar strategy (Gao et al 1989 J. Immunol. 143, 3009-3014), (Townsend et al 1986 Cell 44, 959-968), Nicholas et al 1989 J. Immunol. 143, 2790-2796), Van de Zee et al 1989).

To the best of our knowledge the studies reported here describe the first functional T-helper epitope within the ORF proteins of anogenital HPVs. Basic immunological studies such as those reported here and our previous study (Tindle et al Peptide Research 3, 162-166 1990) are required to lay the foundation of an informed vaccine strategy. DRAHYNI is a Th cell stimulating epitope which can be used for eliciting cognate interaction between T- and B- lymphocytes for the production of antibody against whole E7 protein. Using these criteria DRAHYNI is suitable for inclusion into a synthetic subunit vaccine for anogenital HPV.

The experiments discussed above were all applied to mice. It is now evident from other experiments (ie. the DAY ZERO PROLIFERATIVE ASSAY) that have taken place that peptides containing the DRAHYNI T-epitope elicit proliferation in human subjects of haplotypes DR3, DRW8 and DR2, DRW12. The precise restriction element has as yet not been mapped but it is significant that DR2 covers 26% and DR3 covers 21% of the caucasoid population. While in man the epitope therefore shows greater restriction than in mouse it would still seem to be widely applicable.

It will be appreciated from the foregoing that the peptides of the invention can be made synthetically using standard techniques well known to the skilled chemist. However it should be emphasised that the peptides of the invention can also be produced by recombinant DNA methods as will also be known to the skilled addressee.

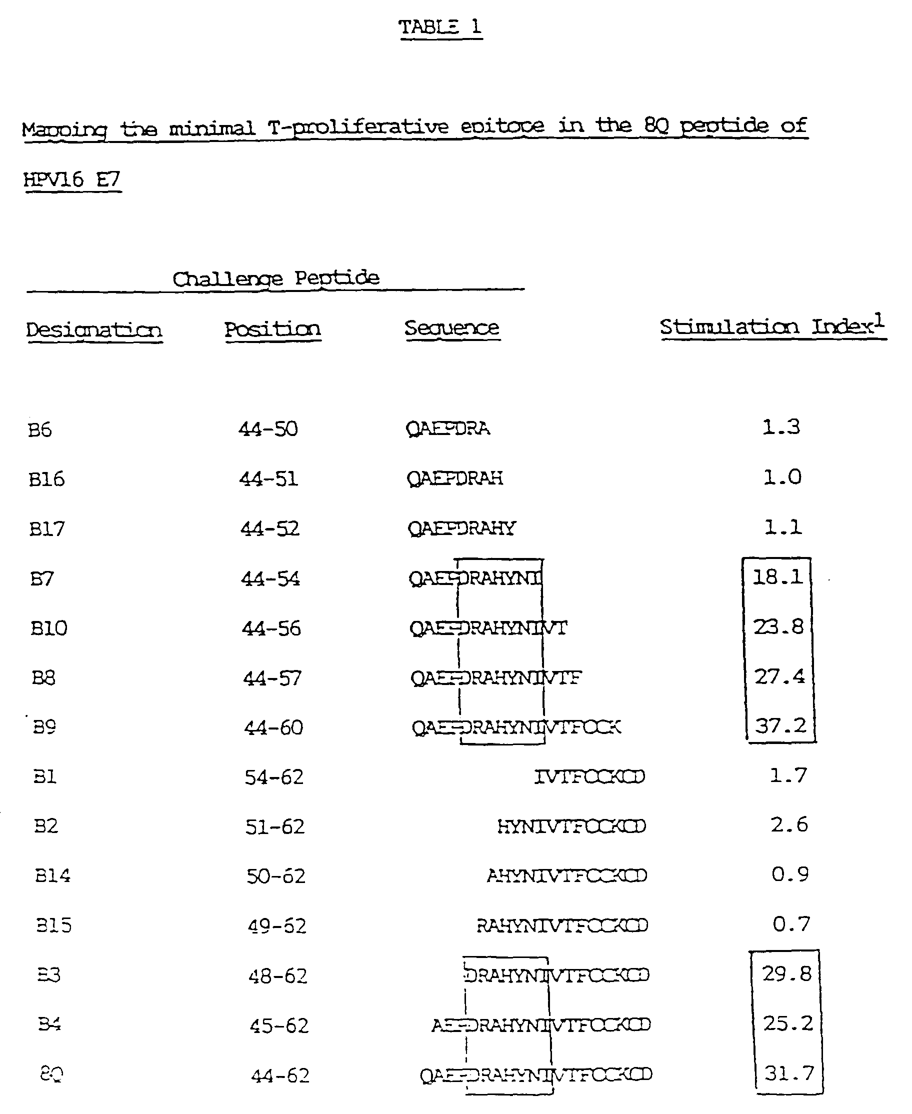

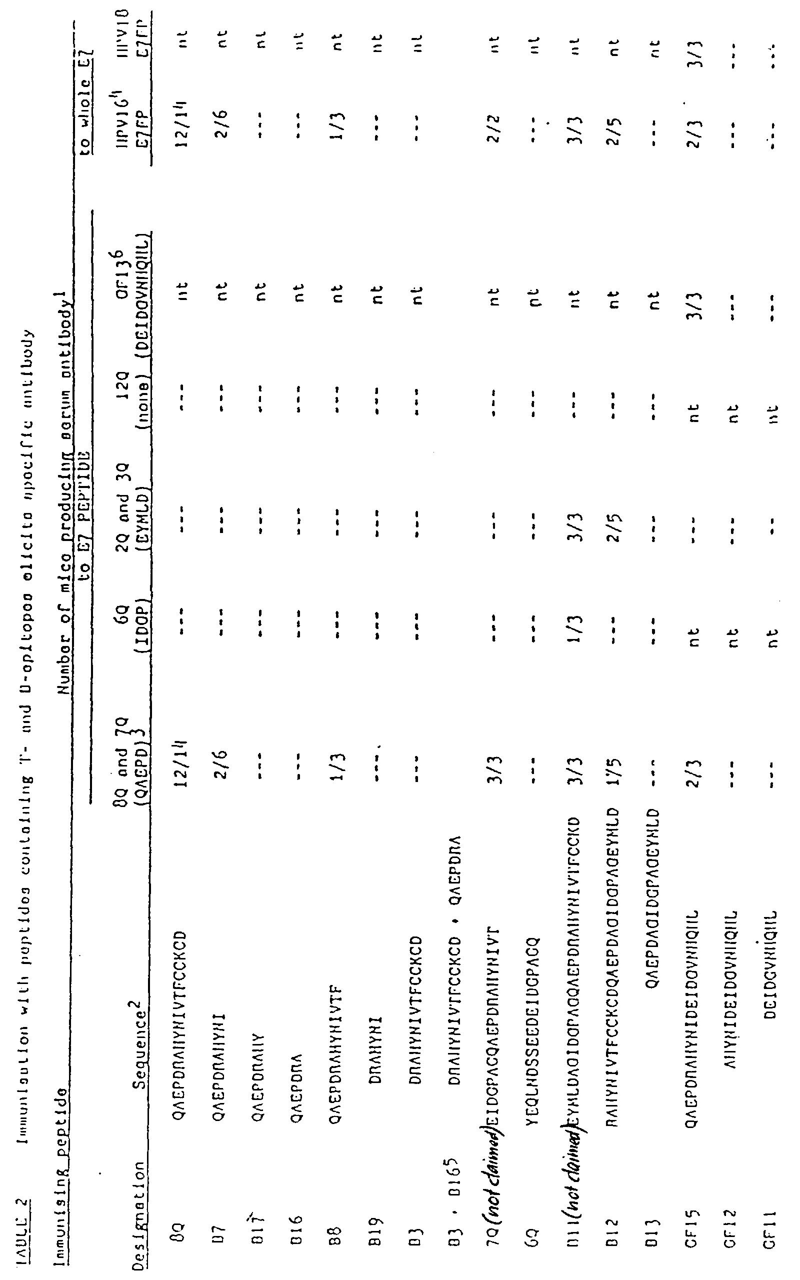

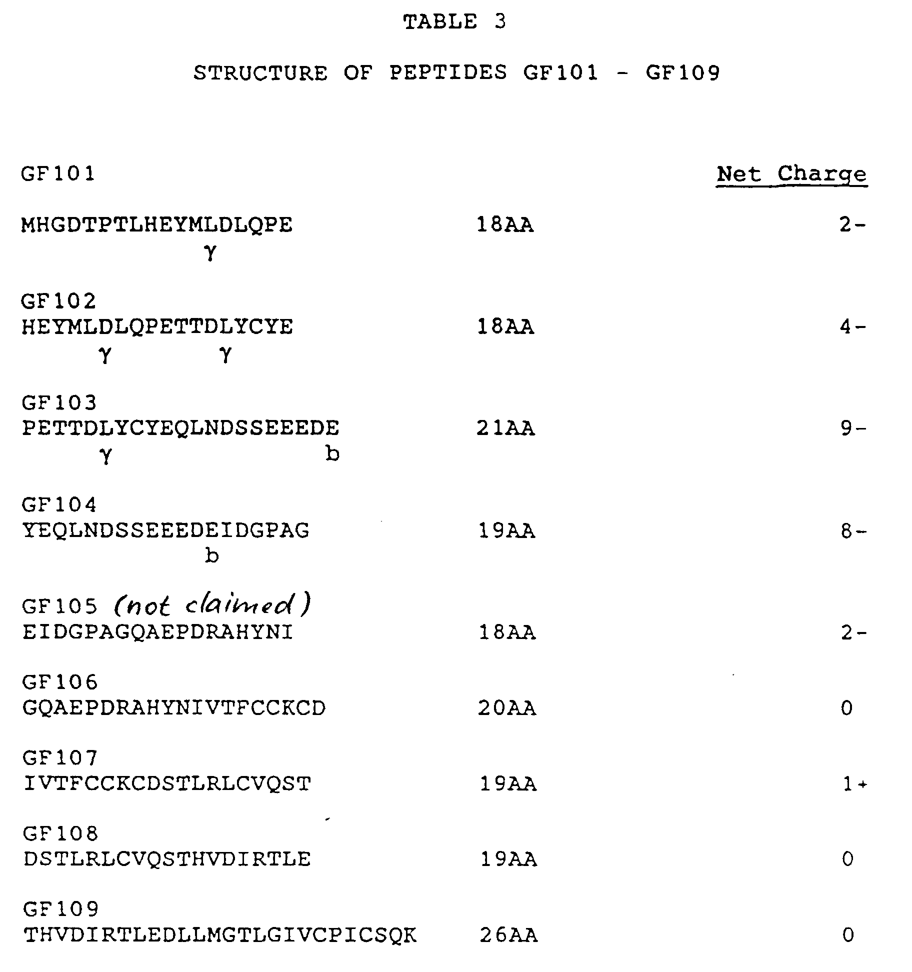

**TABLE 4**

| CONCENTRATIONS | | | | |
|---|---|---|---|---|
| Cells challenged with | | | | |
| 106/8Q | 2 µg/ml | 20 µg/ml | 20 µg/ml + PHA | PHA alone |
| Controls | Tet Tox positive | Media negative | media negativ | |
| 101-103 | 2 µg/ml | 20 µg/ml | 20µg/ml + PHA | |
| 104-106 | 2 µg/ml | 20 µg/ml | 20µg/ml + PHA | |
| 107-109 | 2 µg/ml | 20 µg/ml | 20µg/ml + PHA | |

**TABLE 5**

| STIMULATION INDICED PRODUCED BY CELLS REPEATEDLY STIMULATED WITH VARIOUS PEPTIDE COMBINATIONS | | | | | |
|---|---|---|---|---|---|
| Subject | Peptide Groups 101-103 | 104-106 | 107-109 | 106 | Control Positive Final Stimulation with PHA |
| NOELA DAY | | | | | |
| 0 | 4.8 | 4.1 | - | - | 11.0 |
| DR3 20 | - | - | - | - | 252.1 |
| DR11 27 | - | - | - | - | 67.5 |

| IAN DAY | | | | | |
|---|---|---|---|---|---|
| DR4 0 | 3.6 | 3.7 | - | - | 62.2 |
| DR7 20 | - | - | - | - | 180.2 |
| 27 | - | - | - | - | 10.4 |

| JOE DAY | | | | | |
|---|---|---|---|---|---|
| 0 | - | 2.1 | - | 2.1 | 2.0 |
| DR2 20 | 3.13 | 8.1 | 556.2 | - | 251.6 |
| DRW12 27 | - | - | - | - | 475.7 |

| JULIA DAY | | | | | |
|---|---|---|---|---|---|
| 0 | 2.9 | - | - | - | 1.94 |
| DR1 20 | - | - | 2.26 | - | 229.3 |
| DR4 27 | 2.2 | - | 10.6 | - | 193.1 |

| CHEONG | | | | | |
|---|---|---|---|---|---|
| 0 | 2.1 | - | - | - | 32.5 |
| DR2 20 | - | - | - | - | 671.9 |
| DRW9 NA | - | - | - | - | 165.9 |

| DAVIDSON | | | | | |
|---|---|---|---|---|---|
| 0 | 5.9/4.4** | 7.1/5.3 | 4.3/2.8 | 2.8/2.4 | 20.9 |
| DR3 20 | 9.2 | - | - | 2.6 *** | 31.4 |
| DRW8 NA | 2.0 | - | - | - | 4.3 |

| TREVOR | | | | | |
|---|---|---|---|---|---|
| 0 | - | - | 19.9 | - | 222.8 |
| DR4 20 | - | - | - | - | - |
| DRW6 27 | - | - | - | - | - |

| BRAD | | | | | |
|---|---|---|---|---|---|
| 0 | - | - | - | - | 151.8 |
| DR3 20 | - | - | - | - | - |
| DRW6 27 | - | - | - | - | - |

**Table 6**

| Influence of the antigen delivery system on the humoral immune response to the peptide BT5 | | | | | |
|---|---|---|---|---|---|
| | | | Number of mice with measureable antibody to | | |
| Delivery system* | Antigen# | ug peptide | 7Q | 16E7/MS2 | 18E7/MS2 |
| CFA | BT5 | 100 | 2/2 | 2/2 | 0/2 |
| ISCOM | BT5/LAP20 | 20 | 2/3 | 2/3 | 0/3 |
| ISCOM | BT5 | 20 | 0/2 | 0/2 | 0/2 |
| Saline | BT5/LAP20 | 20 | 0/4 | ND | ND |

| | | | | | |
|---|---|---|---|---|---|
| * Mice immunized on day 0 and day 21, and bled on day 35 | | | | | |
| # See methods No antibodies were detected in the first bleed which was done on day 21. | | | | | |

**Table 7**

| | Peptide used which is treated with glutaraldehyde | | | |
|---|---|---|---|---|
| Total protein at each step | BT5/LAP20 | BT5 | GF23/LAP20 | GF23 |
| 1). After glutaraldehyde treatment and dialysis | 600ug | 600ug | 125ug | 125ug |
| 2). After ISCOM prep. and dialysis, and removal of the precipitate | 334ug | 145ug | 56ug (164ug) | 36ug (153ug) |
| 3). After ultracentifugation to to remove unbound peptide from ISCOMs | 15.6ug | 9.9ug | 15ug | 16ug |

### Legend

### Figure 1

A. The set of overlapping peptides (termed 2Q-12Q) spanning the putative HPV16 E7 protein, used to locate the position of T-proliferative epitopes (see text). Linear B-epitopes defined by monoclonal antibodies 8F, 4F and 10F (Tindle et al 1990) are boxed. Underlining denotes the positions of putative T-epitopes as predicted by Rothbard (r) and DeLisi & Berzofsky (b) algorithms respectively.
B. Lymph Node Cell (LNC) proliferation assay. LNC from C57B1/6 mice immunised with equimolar mixes in CFA of (panel a) peptides 2Q-5Q inclusive, (panel b) peptides 6Q-9Q inclusive and (panel c) peptides 10Q-12Q inclusive (3 mice per group, cells pooled) were challenged in vitro with 20 ug/ml or 2 ug/ml of individual peptides 2Q-12Q. Background (no added Ag.) was 845 ± 90 cpm for 23Q-5Q immunised mice, 1277 ± 330 cpm for 6Q-9Q mice, and 1190 ± 312 for 10Q - 12Q mice, and was subtracted from the results with peptide. Results are shown as arithmetic mean of triplicate wells. A positive control of PPD challenged LNC gave 168, 849 ± 8,346 cpm.

### Legend

### Figure 2

The in vitro proliferative response (panels A-C) and lymphokine production (panel D) of LNC from mice immunised 8 days previously with HPV16 E7 or E7 peptides, and challenged with HPV16 E7 or various E7 peptides.
A. LNC from congenic mice (3 mice per group, cells pooled) immunised with an equimolar mix of peptides 8Q and 6Q were challenged with various concentrations of 8Q (open symbols) or 6Q (closed symbols).
   , B10.D2 (I-A^{dl-E}d); , B10.A (I-A^{a}I-E^{a}); , B10.BR (I-A^{k}I-E^{k});
   , B10.A (2R) (I-A^{k}I-E^{d}); ,B10.A (4R) (I-A^{k}I-E^{b}).
   Background cpm (no added antigen) and PPD response were 2,638 and 88,105 for B10.D2; 7,854 and 75,061 for B10.A; 2.600 and 93,503 for B10.BR; 1,091 and 88,721 for B10.A (2R); and 2,600 and 93,376 for B10.A(4R). No response to peptide 8Q was seen in 'sham'-immunised mice (RPMI plus adjuvant) except in B10.A and B10.A9 (2R) mice which recorded 15,437 and 18,972 cpm. and 5,623 and 8,747 cpm at 8Q peptide concentrations of 9 and 27 ug/ml respectively.
B. LNC from 8Q or 'sham'-immunised B10A(2R) mice (5 per group) were challenged with 16 or 64 ug/ml of HPV16 E7 FP, or 2 or 8 ug/ml of 8Q. The HPV16 E7 and 8Q challenge doses were approximately equimolar for the 8Q sequence. Background controls (no added antigen) and PPD controls were 1269 cpm and 98,775 cpm respectively.
C. B10.A (2R) mice were immunised with 100 ug HPV16 E7 FP or RPMI ('sham') (5 mice per group) and LNC were challenged with 0.1, 1.0 and 10 ug/ml of 8Q. Background controls (no added Ag) and PPD controls were 361 cpm. and 68,872 cpm. respectively for HPV16 E7 FP immunised mice.
D. 4 Groups of 3 B10.A(4R) mice were immunised with 50 ug of equimolar mixtures of peptides 2Q-5Q inc., 6Q-9Q inc., 10Q-12Q inc. or PBS in CFA ('sham'). LNC from each group were pooled and challenged with each of the peptides at 67 and 6.7 ug/ml individually in separate wells (3 wells per concentration per peptide). Culture supernatants were harvested 3 days later and added at 1:2 dilution to HT-2 cells. The HT-2 cells were pulsed 42-44 h. later for 6 h. with ³H-thymidine, harvested and counted. For clarity, only data on LNC from mice primed with peptide mixture 6Q-9Q and challenged with 6Q, 7Q, 8Q and 9Q are shown. (Data not shown: Supernatants of LNCs from mice primed with 2Q-5Q, 10Q-12Q or PBS and challenged with each of the peptides individually failed to stimulate HT-2 cell division). Background, with RPMI added to HT-2 cells in place of supernatants was 1,870 ± 720 cpm.

### Legend

### Figure 3.

Immunisation with peptide 8Q primed mice for in vivo challenge with HPV16 E7 produced from a recombinant vaccinia virus.

Five groups of 6 mice were immunised with 50-100 ug of equimolar mixtures of peptides 2Q-5Q inc., 6Q-9Q inc., 10Q-12Q inc., or 8Q alone, or PBS in CFA ('sham'). 3½ weeks later, each group was divided; 3 mice were challenged with VAC-E7, the other 3 with WR-VAC. Sera were collected after a further 8 and 13 days, and antibody to HPV16.E7 FP and peptides 8Q (containing QAEPD B-epitope), 2Q (containing EYMLD B-epitope), 6Q (containing IDGP B-epitope) or 12Q (containing no B-epitope) was assessed by ELISA. For clarity, only ELISA results on pooled 8 day sera from 8Q primed mice (○,●) and 'sham' primed mice (∇, 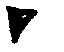 ) challenged with VAC-E7 (○, ●), or WR-VAC (○, 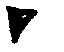 ), on HPV16 E7 Fusion protein (A), or B) peptide 8Q (B) are shown. (Data not shown: Sera from 8Q primed mice challenged with VAC E7 or WR-VAC did not react with 2Q, 6Q or 12Q peptides. Sera from mice primed with 2Q-5Q, or 10-12Q and challenged with VAC E7 or WR-VAC did not react with peptides 2Q, 6Q, 8Q or 12Q or with HPV16 E7 FP. Sera from mice immunised with 6Q-9Q and infected with VAC-E7 or WR-VAC showed a reactivity pattern identical to mice immunised with 8Q alone. Results on 13 day sera were similar to those shown for 8 day sera).

### Legend

### Figure 4.

Sera from B10.A (2R) mice immunised with peptide 8Q (○) or control peptide 3Q (●) in CFA were assayed by ELISA for antibody to (panel A) peptide 8Q, (panel B) peptide 7Q, (panel C) peptide 6Q, (panel D) HPV16 E7 FP. Data points are arithmetic means (± standard deviation) of sera collected individually from 3 mice. (Data not shown: For panels A-C fruther negative controls were 1) lack of reactivity of sera from mice immunised with 2Q, 4Q,10Q and PBS on plates coated with 8Q and 7Q. 2) the lack of reactivity of sera from micr immunised with 8Q on plates coated with peptides 2Q, 4Q and 10Q. For panel D, a further negative control was the lack of reactivity of sera from mice immunised with 8Q on plates coated with HPV16 E6 FP). Peptide 7Q is not claimed.

### Legend

### Table 1.

1. Lymph node cells from B10.A(2R) mice immunised with peptide 8Q (3 mice, cells pooled) were challenged in vitro with peptides as indicated. The cells were cultured for 4 days and proliferation measured by [³H]-thymidine incorporation. The data in this table were pooled from 4 experiments and proliferation is expressed as a stimulation index to normalise for inter-experimental variation. The stimulation index was defined as the ratio of mean cpm of test wells with added antigen, to the mean cpm of test wells with no added antigen. Background cpm (no added Ag) was within the range 3807-5423.

### Legend

### Table 2.

1. Sera from individual mice (at least 3 per group) immunised with the various peptide constructs, were reacted over a range of doubling dilutions 1:64 - 1:40976 with microtitre plates to which were bound peptides 8Q, 7Q, 6Q, 2Q, 3Q, 12Q or GF13, and HPV16 E7 or HPV18 E7, in ELISA assay. '-' indicates no antibody detected by ^{OD}414 readings or >1 and <0.1 respectively at a serum dilution of 1:512 for peptides and by readings of >0.5 and <0.1 respectively at a serum dilution of 1:256 for E7 FP.
2. Sequences QAEPF, IDGP and EYMLD are immunodominant linear B-epitopes in HPV16 E7 proteins (Tindle et al 1990). Sequence DEIDGVNHQHL is an immunodominant B-epitopic region in HPV18 E7 (Selvey et al 1990 J. Immunol. 145, 3105-3110).
3. Sequences in parentheses indicate B-epitopes which the peptides contain.
4. None of the sera reactive with HPV16 E7 FP reacted with HPV16 E6 FP (negative control).
5. Mice were immunised 2-3x ip with 50 ug peptide B3 in CFA at 2 week intervals followed by a final injection of peptide B16. Sera were prepared 3 and 5 days later.
6. The full sequence of GF13 is RAHYNIDEIDGVNHQHL.

SEQUENCE LISTING

(2) INFORMATION FOR SEQ ID NO:1:

(i) SEQUENCE CHARACTERISTICS:
(A) LENGTH: 11 amino acids
(B) TYPE: amino acid
(D) TOPOLOGY: linear

(ii) MOLECULE TYPE: peptide

(xi) SEQUENCE DESCRIPTION: SEQ ID NO:1:

Gln Ala Glu Pro Asp Arg Ala His Tyr Asn Ile
1               5                   10

(2) INFORMATION FOR SEQ ID NO:2:

(i) SEQUENCE CHARACTERISTICS:
(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:2:

Asp Arg Ala His Tyr Asn Ile Asp Gly Pro
1               5                   10

(2) INFORMATION FOR SEQ ID NO:3:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:3:

Gln Ala Glu Pro Asp Arg Ala His Tyr Asn Ile Val Thr Phe Cys Cys
1               5                   10                  15

Lys Cys Asp

(2) INFORMATION FOR SEQ ID NO:4:

(i) SEQUENCE CHARACTERISTICS:
(A) LENGTH: 14 amino acids
(B) TYPE: amino acid
(D) TOPOLOGY: linear

(ii) MOLECULE TYPE: peptide

(xi) SEQUENCE DESCRIPTION: SEQ ID NO:4:

Gln Ala Glu Pro Asp Arg Ala His Tyr Asn Ile Val Thr Phe
1               5                   10

(2) INFORMATION FOR SEQ ID NO:5:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:5:

Glu Ile Asp Gly Pro Ala Gly Gln Ala Glu Pro Asp Arg Ala His Tyr
1               5                   10                  15

Asn Ile Val Thr
            20

(2) INFORMATION FOR SEQ ID NO:6:

(i) SEQUENCE CHARACTERISTICS:
(A) LENGTH: 22 amino acids
(B) TYPE: amino acid
(D) TOPOLOGY: linear

(ii) MOLECULE TYPE: peptide

(xi) SEQUENCE DESCRIPTION: SEQ ID NO:6:

Gln Ala Glu Pro Asp Arg Ala His Tyr Asn Ile Asp Glu Ile Asp Gly
1               5                   10                  15

Val Asn His Gln His Leu
            20

(2) INFORMATION FOR SEQ ID NO:7:

(i) SEQUENCE CHARACTERISTICS:
(A) LENGTH: 32 amino acids
(B) TYPE: amino acid
(D) TOPOLOGY: linear

(ii) MOLECULE TYPE: peptide

(xi) SEQUENCE DESCRIPTION: SEQ ID NO:7:

Glu Tyr Met Leu Asp Ala Gly Ile Asp Gly Pro Ala Gly Gln Ala Glu
1               5                   10                  15

Pro Asp Arg Ala His Tyr Asn Ile Val Thr Phe Cys Cys Lys Cys Asp
            20                      25                      30

(2) INFORMATION FOR SEQ ID NO:8:

     (i) SEQUENCE CHARACTERISTICS:
          (A) LENGTH: 32 amino acids
          (B) TYPE: amino acid
          (D) TOPOLOGY: linear

    (ii) MOLECULE TYPE: peptide

    (xi) SEQUENCE DESCRIPTION: SEQ ID NO:8:

     Arg Ala His Tyr Asn Ile Val Thr Phe Cys Cys Lys Cys Asp Gln Ala
     1               5                   10                  15

     Glu Pro Asp Ala Gly Ile Asp Gly Pro Ala Gly Glu Tyr Met Leu Asp
                 20                  25                  30

(2) INFORMATION FOR SEQ ID NO:9:

     (i) SEQUENCE CHARACTERISTICS:
          (A) LENGTH: 18 amino acids
          (B) TYPE: amino acid
          (D) TOPOLOGY: linear

    (ii) MOLECULE TYPE: peptide

    (xi) SEQUENCE DESCRIPTION: SEQ ID NO:9:

     Glu Ile Asp Gly Pro Ala Gly Gln Ala Glu Pro Asp Arg Ala His Tyr
     1               5                   10                  15

     Asn Ile

(2) INFORMATION FOR SEQ ID NO:10:

     (i) SEQUENCE CHARACTERISTICS:
          (A) LENGTH: 20 amino acids
          (B) TYPE: amino acid
          (D) TOPOLOGY: linear

    (ii) MOLECULE TYPE: peptide

    (xi) SEQUENCE DESCRIPTION: SEQ ID NO:10:

     Gly Gln Ala Glu Pro Asp Arg Ala His Tyr Asn Ile Val Thr Phe Cys
     1               5                   10                  15

Cys Lys Cys Asp
            20

(2) INFORMATION FOR SEQ ID NO:11:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:11:

Asp Arg Ala His Tyr Asn Ile
1               5

(2) INFORMATION FOR SEQ ID NO:12:

(i) SEQUENCE CHARACTERISTICS:
(A) LENGTH: 5 amino acids
(B) TYPE: amino acid
(D) TOPOLOGY: linear

(ii) MOLECULE TYPE: peptide

(xi) SEQUENCE DESCRIPTION: SEQ ID NO:12:

Gln Ala Glu Pro Asp
1               5

(2) INFORMATION FOR SEQ ID NO:13:

(i) SEQUENCE CHARACTERISTICS:
(A) LENGTH: 4 amino acids
(B) TYPE: amino acid
(D) TOPOLOGY: linear

(ii) MOLECULE TYPE: peptide

(xi) SEQUENCE DESCRIPTION: SEQ ID NO:13:

Ile Asp Gly Pro
1

(2) INFORMATION FOR SEQ ID NO:14:

(i) SEQUENCE CHARACTERISTICS:
(A) LENGTH: 5 amino acids
(B) TYPE: amino acid
(D) TOPOLOGY: linear

(ii) MOLECULE TYPE: peptide

(xi) SEQUENCE DESCRIPTION: SEQ ID NO:14:

Glu Tyr Met Leu Asp
1               5

(2) INFORMATION FOR SEQ ID NO:15:

(i) SEQUENCE CHARACTERISTICS:
(A) LENGTH: 4 amino acids
(B) TYPE: amino acid
(D) TOPOLOGY: linear

(ii) MOLECULE TYPE: peptide

(xi) SEQUENCE DESCRIPTION: SEQ ID NO:15:

Tyr Met Leu Asp
1

(2) INFORMATION FOR SEQ ID NO:16:

(i) SEQUENCE CHARACTERISTICS:
(A) LENGTH: 11 amino acids
(B) TYPE: amino acid
(D) TOPOLOGY: linear

(ii) MOLECULE TYPE: peptide

(xi) SEQUENCE DESCRIPTION: SEQ ID NO:16:

Asp Glu Ile Asp Gly Val Asn His Gln His Leu
1               5                   10

(2) INFORMATION FOR SEQ ID NO:17:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:17:

Ser Glu Glu Asn Glu Asp
1               5

(2) INFORMATION FOR SEQ ID NO:18:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:18:

Asp Arg Ala His Tyr Asn Ile Val Thr Phe Cys Cys Lys Cys Asp
1               5                   10                  15

(2) INFORMATION FOR SEQ ID NO:19:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:19:

Val Ser Ser Leu Leu Ser Ser Leu Lys Glu Tyr Trp Ser Ser Leu Lys
1               5                   10                  15

Glu Ser Phe Ser
            20

(2) INFORMATION FOR SEQ ID NO:20:

(i) SEQUENCE CHARACTERISTICS:
(A) LENGTH: 26 amino acids
(B) TYPE: amino acid
(D) TOPOLOGY: linear

(ii) MOLECULE TYPE: peptide

(xi) SEQUENCE DESCRIPTION: SEQ ID NO:20:

Thr Arg Lys Ser Ile Arg Ile Gln Arg Gly Pro Asp Arg Ala His Tyr
1               5                   10                  15

Asn Ile Val Thr Phe Cys Cys Lys Cys Asp
            20                  25

(2) INFORMATION FOR SEQ ID NO:21:

(i) SEQUENCE CHARACTERISTICS:
(A) LENGTH: 7 amino acids
(B) TYPE: amino acid
(D) TOPOLOGY: linear

(ii) MOLECULE TYPE: peptide

(xi) SEQUENCE DESCRIPTION: SEQ ID NO:21:

Gln Ala Glu Pro Asp Arg Ala
1               5

(2) INFORMATION FOR SEQ ID NO:22:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:22:

Gln Ala Glu Pro Asp Arg Ala His
1               5

(2) INFORMATION FOR SEQ ID NO:23:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:23:

Gln Ala Glu Pro Asp Arg Ala His Tyr
1               5

(2) INFORMATION FOR SEQ ID NO:24:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:24:

Gln Ala Glu Pro Asp Arg Ala His Tyr Asn Ile Val Thr
1               5                   10

(2) INFORMATION FOR SEQ ID NO:25:

(i) SEQUENCE CHARACTERISTICS:
(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO:25:

Gln Ala Glu Pro Asp Arg Ala His Tyr Asn Ile Val Thr Phe Cys Cys
1               5                   10                  15

Lys

(2) INFORMATION FOR SEQ ID NO:26:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:26:

Ile Val Thr Phe Cys Cys Lys Cys Asp
1               5

(2) INFORMATION FOR SEQ ID NO:27:

(i) SEQUENCE CHARACTERISTICS:
(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:27:

His Tyr Asn Ile Val Thr Phe Cys Cys Lys Cys Asp
1               5                   10

'2) INFORMATION FOR SEQ ID NO:28:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:28:

Ala His Tyr Asn Ile Val Thr Phe Cys Cys Lys Cys Asp
1               5                   10

(2) INFORMATION FOR SEQ ID NO:29:

(i) SEQUENCE CHARACTERISTICS:
(A) LENGTH: 14 amino acids
(B) TYPE: amino acid
(D) TOPOLOGY: linear

(ii) MOLECULE TYPE: peptide

(xi) SEQUENCE DESCRIPTION: SEQ ID NO:29:

Arg Ala His Tyr Asn Ile Val Thr Phe Cys Cys Lys Cys Asp
1               5                   10

(2) INFORMATION FOR SEQ ID NO:30:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:30:

Ala Glu Pro Asp Arg Ala His Tyr Asn Ile Val Thr Phe Cys Cys Lys
1               5                   10                  15

Cys Asp

(2) INFORMATION FOR SEQ ID NO:31:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:31:

Tyr Glu Gln Leu Asn Asp Ser Ser Glu Glu Glu Asp Glu Ile Asp Gly
1               5                   10                  15

Pro Ala Gly Gln
            20

(2) INFORMATION FOR SEQ ID NO:32:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:32:

Gln Ala Glu Pro Asp Ala Gly Ile Asp Gly Pro Ala Gly Glu Tyr Met
1               5                   10                  15

Leu Asp

(2) INFORMATION FOR SEQ ID NO:33:

(i) SEQUENCE CHARACTERISTICS:
(A) LENGTH: 16 amino acids
(B) TYPE: amino acid
(D) TOPOLOGY: linear

(ii) MOLECULE TYPE: peptide

(xi) SEQUENCE DESCRIPTION: SEQ ID NO:33:

Ala His Tyr Asn Ile Asp Glu Ile Asp Gly Val Asn His Gln His Leu
1               5                   10                  15

(2) INFORMATION FOR SEQ ID NO:34:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:34:

Met His Gly Asp Thr Pro Thr Leu His Glu Tyr Met Leu Asp Leu Gln
1               5                   10                  15

Pro Glu

(2) INFORMATION FOR SEQ ID NO:35:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:35:

His Glu Tyr Met Leu Asp Leu Gln Pro Glu Thr Thr Asp Leu Tyr Cys
1               5                   10                  15

Tyr Glu

(2) INFORMATION FOR SEQ ID NO:36:

(i) SEQUENCE CHARACTERISTICS:
(A) LENGTH: 21 amino acids
(B) TYPE: amino acid
(D) TOPOLOGY: linear

(ii) MOLECULE TYPE: peptide

(xi) SEQUENCE DESCRIPTION: SEQ ID NO:36:

Pro Glu Thr Thr Asp Leu Tyr Cys Tyr Glu Gln Leu Asn Asp Ser Ser
1               5                   10                  15

Glu Glu Glu Asp Glu
            20

(2) INFORMATION FOR SEQ ID NO:37:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:37:

Tyr Glu Gln Leu Asn Asp Ser Ser Glu Glu Glu Asp Glu Ile Asp Gly
1               5                   10                  15

Pro Ala Gly

(2) INFORMATION FOR SEQ ID NO:38:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:38:

Ile Val Thr Phe Cys Cys Lys Cys Asp Ser Thr Leu Arg Leu Cys Val
1               5                   10                  15

Gln Ser Thr

(2) INFORMATION FOR SEQ ID NO:39:

(i) SEQUENCE CHARACTERISTICS:

(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:39:

Asp Ser Thr Leu Arg Leu Cys Val Gln Ser Thr His Val Asp Ile Arg
1               5                   10                  15

Thr Leu Glu

(2) INFORMATION FOR SEQ ID NO:40:

(i) SEQUENCE CHARACTERISTICS:
(A) LENGTH: 26 amino acids
(B) TYPE: amino acid
(D) TOPOLOGY: linear

(ii) MOLECULE TYPE: peptide

(xi) SEQUENCE DESCRIPTION: SEQ ID NO:40:

Thr His Val Asp Ile Arg Thr Leu Glu Asp Leu Leu Met Gly Thr Leu
1               5                   10                  15

Gly Ile Val Cys Pro Ile Cys Ser Gln Lys
            20                  25

(2) INFORMATION FOR SEQ ID NO:41:

(i) SEQUENCE CHARACTERISTICS:
(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO:41:

Arg Ala His Tyr Asn Ile Asp Glu Ile Asp Gly Val Asn His Gln His
1               5                   10                  15

Leu

(2) INFORMATION FOR SEQ ID NO:42:

(i) SEQUENCE CHARACTERISTICS:
(A) LENGTH: 98 amino acids
(B) TYPE: amino acid
(D) TOPOLOGY: linear

(ii) MOLECULE TYPE: peptide

(xi) SEQUENCE DESCRIPTION: SEQ ID NO:42:

Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp
        35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

Lys Pro

(2) INFORMATION FOR SEQ ID NO:43:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:43:

Met His Gly Asp Thr Pro Thr Leu His Glu Tyr Met Leu Asp Leu Gln
1               5                   10                  15

Pro Glu Thr Thr
            20

(2) INFORMATION FOR SEQ ID NO:44:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:44:

Pro Thr Leu His Glu Tyr Met Leu Asp Leu Gln Pro Glu Thr Thr Asp
1               5                   10                  15

Leu Tyr Cys Tyr
            20

(2) INFORMATION FOR SEQ ID NO:45:

    (i) SEQUENCE CHARACTERISTICS:
        (A) LENGTH: 18 amino acids
        (B) TYPE: amino acid
        (D) TOPOLOGY: linear

    (ii) MOLECULE TYPE: peptide

    (xi) SEQUENCE DESCRIPTION: SEQ ID NO:45:

Met Leu Asp Leu Gln Pro Glu Thr Thr Asp Leu Tyr Cys Tyr Glu Gln
1               5                   10                  15

Leu Asn

(2) INFORMATION FOR SEQ ID NO:46:

    (i) SEQUENCE CHARACTERISTICS:
        (A) LENGTH: 20 amino acids
        (B) TYPE: amino acid
        (D) TOPOLOGY: linear

    (ii) MOLECULE TYPE: peptide

    (xi) SEQUENCE DESCRIPTION: SEQ ID NO:46:

Glu Thr Thr Asp Leu Tyr Cys Tyr Glu Gln Leu Asn Asp Ser Ser Glu
1               5                   10                  15

Glu Glu Asp Glu
            20

(2) INFORMATION FOR SEQ ID NO:47:

    (i) SEQUENCE CHARACTERISTICS:
        (A) LENGTH: 25 amino acids
        (B) TYPE: amino acid
        (D) TOPOLOGY: linear

    (ii) MOLECULE TYPE: peptide

    (xi) SEQUENCE DESCRIPTION: SEQ ID NO:47:

Ala His Tyr Asn Ile Val Thr Phe Cys Cys Lys Cys Asp Ser Thr Leu
1               5                   10                  15

Arg Leu Cys Val  Gln Ser Thr His Val
            20                   25

(2) INFORMATION FOR SEQ ID NO:48:

     (i) SEQUENCE CHARACTERISTICS:
          (A) LENGTH: 17 amino acids
          (B) TYPE: amino acid
          (D) TOPOLOGY: linear

    (ii) MOLECULE TYPE: peptide

    (xi) SEQUENCE DESCRIPTION: SEQ ID NO:48:

     Ser Thr Leu Arg Leu Cys Val Gln Ser Thr His Val Asp Ile Arg Thr
     1               5                   10                  15

     Leu

(2) INFORMATION FOR SEQ ID NO:49:

     (i) SEQUENCE CHARACTERISTICS:
          (A) LENGTH: 20 amino acids
          (B) TYPE: amino acid
          (D) TOPOLOGY: linear

    (ii) MOLECULE TYPE: peptide

    (xi) SEQUENCE DESCRIPTION: SEQ ID NO:49:

     Gln Ser Thr His Val Asp Ile Arg Thr Leu Glu Asp Leu Leu Met Gly
     1               5                   10                  15

     Thr Leu Gly Ile
                 20

(2) INFORMATION FOR SEQ ID NO:50:

     (i) SEQUENCE CHARACTERISTICS:
          (A) LENGTH: 20 amino acids
          (B) TYPE: amino acid
          (D) TOPOLOGY: linear

    (ii) MOLECULE TYPE: peptide

    (xi) SEQUENCE DESCRIPTION: SEQ ID NO:50:

     Asp Ile Arg Thr Leu Glu Asp Leu Leu Met Gly Thr Leu Gly Ile Val
     1               5                   10                  15

     Cys Pro Ile Cys
                 20

## Claims

1. An HPV vaccine which comprises an immunogenic peptide in combination with a suitable adjuvant, said immunogenic peptide comprising the minimal T helper cell proliferative epitope DRAHYNI (SEQ ID NO: 11), corresponding to residues 48-54 of the HPV16 E7 ORF, which immunogenic peptide further comprises one or more amino acid sequences which correspond to B cell epitopes of HPV16 E7 ORF or HPV18 E7 ORF, and which immunogenic peptide specifically excludes the peptides including GPAGQAEPDRAHYNI.

2. An HPV vaccine according to claim 1, wherein the B cell epitopes are selected from the amino acid sequences QAEPD (SEQ ID NO: 12), IDGP (SEQ ID NO: 13), EYMLD (SEQ ID NO: 14), YMLD (SEQ ID NO: 15) and DEIDGVNHQHL.

3. An HPV vaccine according to claims 1 or 2, wherein a terminal amino acid residue of the B cell epitope is shared with a terminal amino acid residue of the T helper cell proliferative epitope.

4. An HPV vaccine according to claim 1, wherein the immunogenic peptide comprises an amino acid sequence selected from:
QAEPDRAHYNI (SEQ ID NO: 1),
QAEPDRAHYNIVTFCCKCD (SEQ ID NO: 3),
QAEPDRAHYNIVTF (SEQ ID NO: 4),
QAEPDRAHYNIDEIDGVNHQHL (SEQ ID NO: 6),
QAEPDRAHYNIVT (SEQ ID NO:24),
QAEPDRAHYNIVTFCCK (SEQ ID NO:25).

## Patentansprüche

1. HPV-Impfstoff, der ein immunogenes Peptid kombiniert mit einem geeigneten Adjuvans umfaßt, wobei das immunogene Peptid das minimale proliferative T-Helferzell-Epitop DRAHYNI (Seq. Nr.: 11) umfaßt, das den Resten 48-54 des HPV16 E7 ORF entspricht und das immunogene Peptid weiter eine oder mehrere Aminosäuresequenzen umfaßt, die B-Zell-Epitopen des HPV16 E7 ORF oder des HPV18 E7 ORF entsprechen, und wobei das immunogene Peptid insbesondere die Peptide ausschließt, die GPAGQAEPDRAHYNI enthalten.

2. HPV-Impfstoff nach Anspruch 1, bei dem die B-Zell-Epitope aus den Aminosäuresequenzen QAEPD (Seq. Nr.: 12), IDGP (Seq. Nr.: 13), EYMLD (Seq. Nr.:14), YMLD (Seq. Nr.: 15) und DEIDGVNHQHL ausgewählt sind.

3. HPV-Impfstoff nach Anspruch 1 oder 2, bei dem ein endständiger Aminosäurerest des B-Zell-Epitopes einem endständigen Aminosäurerest des proliferativen T-Helferzellepitopes gemeinsam ist.

4. HPV-Impfstoff nach Anspruch 1, bei dem das immunogene Peptid eine Aminosäuresequenz umfaßt, die ausgewählt ist aus:
QAEPDRAHYNI (Seq. Nr.: 1),
QAEPDRAHYNIVTFCCKCD (Seq. Nr.: 3),
QAEPDRAHYNIVTF (Seq. Nr.: 4),
QAEPDRAHYNIDEIDGVNHQHL (Seq. Nr.: 6),
QAEPDRAHYNIVT (Seq. Nr.: 24),
QAEPDRAHYNIVTFCCK (Seq. Nr.: 25).

## Revendications

1. Vaccin contre HPV qui comprend un peptide immunogène en combinaison avec un adjuvant approprié, ledit peptide immunogène comprenant l'épitope minimal prolifératif de lymphocytes T auxiliaires DRAHYNI (SEQ ID NO:11), correspondant aux résidus 48 à 54 de l'ORF E7 de HPV16, lequel peptide immunogène comprend en outre une ou plusieurs séquences d'acides aminés qui correspondent aux épitopes de lymphocytes B de l'ORF E7 de HPV16 ou de l'ORF E7 de HPV18 et, lequel peptide immunogène exclut spécifiquement les peptides incluant GPAGQAEPDRAHYNI.

2. Vaccin contre HPV selon la revendication 1, dans lequel les épitopes de lymphocytes B sont choisis parmi les séquences d'acides aminés QAEPD (SEQ ID NO: 12), IDPG (SEQ ID NO: 13), EYMLD (SEQ ID NO: 14), YMLD (SEQ ID NO: 15) et DEIDGVNHQHL.

3. Vaccin contre HPV selon les revendications 1 ou 2, dans lequel un résidu d'acide aminé terminal de l'épitope de lymphocytes B est commun avec un résidu d'acide aminé terminal de l'épitope prolifératif de lymphocytes T auxiliaires.

4. Vaccin contre HPV selon la revendication 1, dans lequel le peptide immunogène comprend une séquence d'acides aminés choisie parmi :
QAEPDRAHYNI (SEQ ID NO: 1),
QAEPDRAHYNIVTFCCKCD (SEQ ID NO: 3),
QAEPDRAHYNIVTF (SEQ ID NO:4),
QAEPDRAHYNIDEIDGVNHQHL (SEQ ID NO: 6),
QAEPDRAHYNIVT (SEQ ID NO:24),
QAEPDRAHYNIVTFCCK (SEQ ID NO:25).
